# FASCICULE DE BREVET EUROPEEN

(11) **EP 0 532 689 B1**
(45) Date de publication et mention de la délivrance du brevet: **09.03.1994**
(21) Numéro de dépôt: 91912708.4
(22) Date de dépôt: 07.06.1991
(51) Int. Cl.: A61F 2/60, A61F 2/80

(54) **DISPOSITIF DE MISE EN PLACE DES PROTHESES**
EINRICHTUNG ZUM ANLEGEN VON PROTHESEN
PROSTHESIS FITTING DEVICE

(30) Priorité: 07.06.1990 FR 9007452
(43) Date de publication de la demande: 24.03.1993
(73) Titulaire: SARAZIN, Maurice, F-34400 Lunel (FR)
(72) Inventeur: SARAZIN, Maurice, F-34400 Lunel (FR)
(74) Mandataire: Foldes, Georges
(86) Numéro de dépôt international: FR9100453
(87) Numéro de publication internationale: WO9118564

(56) Documents cités:
- DE-C- 806 981
- DE-U- 8 514 423
- FR-A- 2 506 150
- US-A- 3 922 727

## Description

La présente invention concerne un dispositif permettant à un amputé, d'un membre inférieur ou supérieur, de procéder lui-même, et sans effort important, à la mise en place de sa prothèse de contact, ou à faciliter la tâche du personnel hospitalier des établissements spécialisés dans le domaine de la rééducation des amputés.

Dans la pratique courante, la mise en place de telles prothèses, requiert la mise en place préalable d'un fourreau de tissus de jersey, que l'on enfile sur le moignon à appareiller, comme une chaussette. Ce fourreau est nettement plus long que le moignon, et sa partie libre est enfilée, par l'intérieur de l'emboîture de la prothèse, au travers de l'orifice de la soupape d'extraction d'air qui se trouve disposée sur l'extrémité fermée de l'emboîture, au plus près de l'axe longitudinal de celle-ci. Ensuite, l'amputé ou plus souvent une tierce personne, tire sur la partie libre du fourreau de jersey, dépassant à l'extérieur de l'emboîture, pour rétreindre les chairs du moignon, puis le faire pénétrer dans l'emboiture, et in fine, extraire complètement le fourreau de jersey à l'extérieur de la prothèse, en le faisant glisser entre la chair du moignon et la paroi interne de l'emboîture.

Cette traction peut s'effectuer directement à la main ou par l'intermédiaire d'un dispositif du type comportant une canne munie à son extrémité d'un moyen de préhension de la partie libre du fourreau sur laquelle on doit exercer la traction comme c'est le cas du dispositif décrit au brevet DE-U-8 514 423.

Les inconvénients de ce procédé de mise en place sont les suivants :
- l'effort de traction à exercer sur la partie libre du fourreau de jersey est loin d'être négligeable et s'avère déjà assez pénible pour une tierce personne normalement constituée. Cet effort devient très pénible, voir impossible à réaliser pour un amputé surtout s'il est âgé ou fatigué par son handicap, ou s'il a des difficultés à se servir de ses mains.
- cet effort de traction, très important, exige une préhension très énergique du fourreau de jersey par la main de l'opérateur, avec souvent la nécessité de faire un tour mort autour de la main pour mener à bien cette opération. Cela est très éprouvant notamment pour le personnel du corps sanitaire, qui doit plusieurs fois par jour répéter cette opération durant les séances de rééducation. Très souvent, pour les cas difficiles il y a lieu même, de tirer préférentiellement sur telle ou telle autre partie de la circonférence du fourreau de jersey pour privilégier le glissement de celui-ci au niveau des génératrices en contact avec les chairs les plus molles.
- à noter également que le sens dans lequel doit être exercé l'effort de traction est, au point de vue ergonomique, peu compatible avec la morphologie humaine. En effet il est plus facile de faire un effort de traction en tirant vers soi, qu'inversement. C'est pourquoi certains ont proposé un dispositif avec poulie de renvoi et mouflage, prenant appui au niveau du sol sous le pied valide d'un amputé d'un membre inférieur, de façon à inverser le sens de cet effort tout en le démultipliant (voir par exemple le brevet FR 2.506.150). Un tel système s'est peu développé car il s'applique à des cas particuliers et ne résoud pas le problème du guidage de la prothèse durant sa mise en place.

Il en est de même pour les dispositifs du type comportant un moteur reposant au sol dont la commande est effectuée par l'amputé et autour de l'axe duquel peut s'enrouler ou se dérouler ledit fourreau de tissus de jersey comme cela est décrit dans le brevet US 3.922.727.

En effet durant l'opération de mise en place de la prothèse, comme exposé ci-avant, il y a lieu également de retenir la prothèse et de la guider vers le moignon à appareiller, car les forces de frottement exercées par le passage du fourreau de jersey dans l'orifice de la soupage d'extraction d'air, ont plutôt tendance à éloigner l'emboîture du moignon plutôt que de rapprocher ceux-ci.

La présente invention prétend remédier aux inconvénients exposés ci-avant en ce que le dispositif qu'elle a pour objet comporte un moyen démultiplicateur d'effort permettant d'exercer une force de traction très importante, progressive et contrôlable, par effet sensitif, sur le fourreau de jersey et en ce que le dispositif comporte un moyen de préhension du fourreau de jersey permettant de tirer celui-ci, indifféremment sur la totalité de son pourtour ou seulement sur une partie privilégiée de celui-ci, et en ce que le dispositif comporte un moyen permettant de prendre appui au niveau de l'extrémité fermée de la prothèse autour de l'orifice de la soupape d'extraction d'air, et en ce que la réaction de la force de traction s'exerce sur ce point d'appui, ce qui a pour effet de pousser l'emboîture autour du moignon et de la guider en même temps, et en ce que l'effort à exercer manuellement par l'opérateur s'effectue d'une seule main et par simple fermeture des doigts sur une poignée à deux branches, similaire à celle bien connue, des freins de bicyclette, ce qui permet à une personne ne disposant que d'une seule main de positionner le dispositif et de le faire fonctionner sans effort.

Ainsi, selon l'invention ce dispositif de traction sur les fourreaux de jersey pour la mise en place des prothèses de contact qui comprennent une emboîture fermée à l'une de ses extrémités sur laquelle est prévu un orifice d'une soupape d'extraction d'air, ledit dispositif comportant une butée, est caractérisé en ce que cette butée est adaptée à prendre appui sur ladite extrémité fermée de l'emboîture de la prothèse, au voisinage dudit orifice de la soupape d'extraction d'air, sur l'emboîture de façon que la réaction de la force de traction s'exerce sur ce point d'appui de la butée sur ladite extrémité.

Dans la description qui va suivre, le moyen démultiplicateur d'effort est similaire à celui, bien connu, qui est utilisé dans les pistolets distributeurs de produits pâteux (colles ou joint d'étanchéité) produits qui sont vendus conditionnés dans des cartouches tubulaires jetables, comportant un piston en plastique et un embout de distribution vissable. De même, le moyen de préhension du fourreau de jersey est similaire dans son principe à celui des taquets auto-coinceurs, bien connus, que l'on rencontre sur les plats-bords des embarcations à voiles pour y coincer les écoutes. Ces choix, effectués dans un but explicatif et de clarté des dessins annexés, ne préjugent pas de ceux qui seront effectués pour la réalisation effective du dispositif, conformément à l'invention comme revendiquée ci-dessous.

La description qui va suivre, faite en regard des dessins annexés, dans un but explicatif et nullement limitatif, permet de mieux comprendre les avantages, buts et caractéristiques de l'invention.
- Figure 1 représente, en coupe, une emboîture schématisée d'une prothèse de contact. Cette figure ne fait pas partie de l'invention elle n'est donnée que pour mieux faire comprendre avec quels éléments le dispositif, objet de la présente invention, se trouvera en contact lors de son fonctionnement.
- Figure 2 représente, en coupe partielle, une vue d'ensemble du dispositif, pour donner un aperçu de son architecture et la position relative de ses constituants.
- Figure 3 représente, une coupe de chariot de traction du dispositif, selon un plan de coupe perpendiculaire à l'axe longitunal du dispositif, ainsi que le taquet auto-coinçeur.
- Figure 4 représente une vue de dessus du chariot et du taquet auto-coinçeur.
- Figure 5 représente une élévation en coupe partielle de la butée orientable.
- Figure 6 représente une vue de dessus de la butée orientable
- Figure 7 représente, en coupe schématique partielle, le dispositif, appliqué sur une emboiture de prothèse, et cela en début de fonctionnement du dispositif.
- Figure 8 représente les mêmes éléments que la précédente, mais en fin de fonctionnement du dispositif, juste avant que le fourreau de jersey n'échappe de l'extrémité du moignon pour sortir complètement de l'emboîture.
- Figure 9 représente une variante de réalisation du dispositif, où l'effort de traction est obtenu par un moteur électrique similaire à ceux des tournevis électriques.
- La figure 10 représente une première variante de réalisation du dispositif, en fin d'opération d'extraction du fourreau de jersey 1B.
- La figure 11 représente une deuxième variante de réalisation du dispositif, en fin d'opération d'extraction du fourreau de jersey 1B.

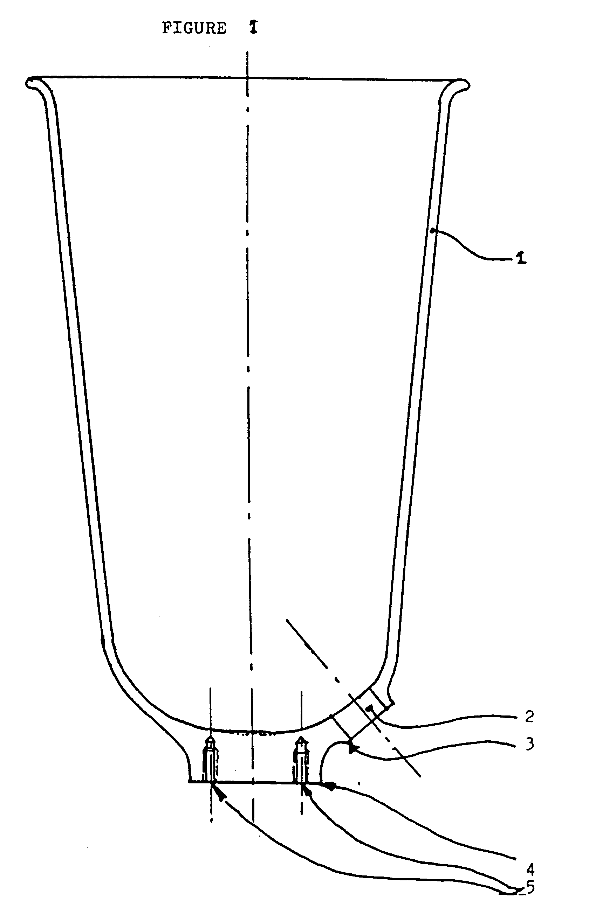

Dans la figure 1 on trouve : un corps d'emboîture 1 comportant un orifice de soupape d'extraction d'air 2 destiné à recevoir une soupape amovible non représentée, et qui est composée d'un bouchon en caoutchouc adapté à s'emboîter sur cet orifice 2, le bouchon étant lui-même équipé d'une soupape adaptée à laisser sortir l'air de l'emboîture 1, durant l'utilisation de la prothèse. L'orifice 2 sert également de passage au fourreau de jersey pour permettre le tirage de celui-ci par l'extérieur de l'emboîture de la prothèse 1, l'orifice 2 débouche, à l'extérieur de l'emboîture 1, sur un bossage de renfort présentant un méplat en forme de couronne circulaire 3.

L'emboîture 1 se termine, à sa partie inférieure, par un plan de pose 4 disposé perpendiculairement à l'axe de l'emboîture 1, destiné à recevoir l'articulation artificielle de la prothèse qui se fixe par des vis, dans les trous taraudés 5. La position obligatoirement centrée du plan de pose 4, explique pourquoi l'orifice de la soupape 2 est excentré et en oblique par rapport à l'axe de l'emboîture.

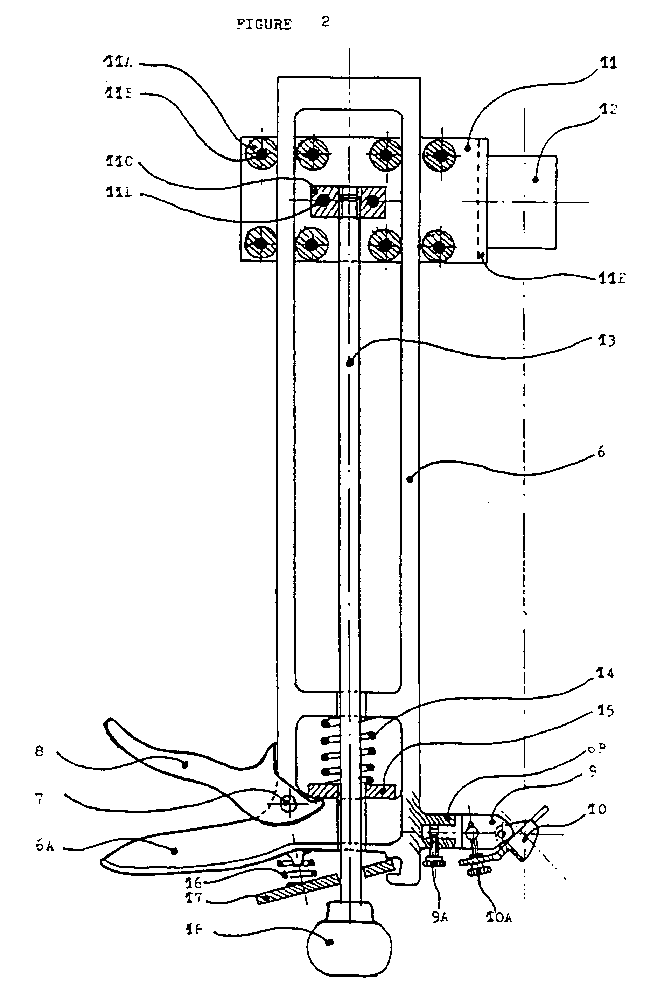

Dans la figure 2 on trouve : un bâti glissière 6, une poignée fixe 6 A, une crapaudine porte butée 6B, un axe de poignée mobile 7, une poignée mobile 8, une tourelle porte butée 9, une vis 9A, une butée orientable 10, une vis 10A, un chariot de traction 11, formé de deux flasques 11E, huit galets 11A, huit axes 11B, une entretoise 11C, deux rivets 11D, un taquet auto-coinçeur 12, une tige de poussée 13, un ressort 14, un linguet de poussée 15, un ressort 16, un linguet de retenue 17, un bouton de rappel 18.

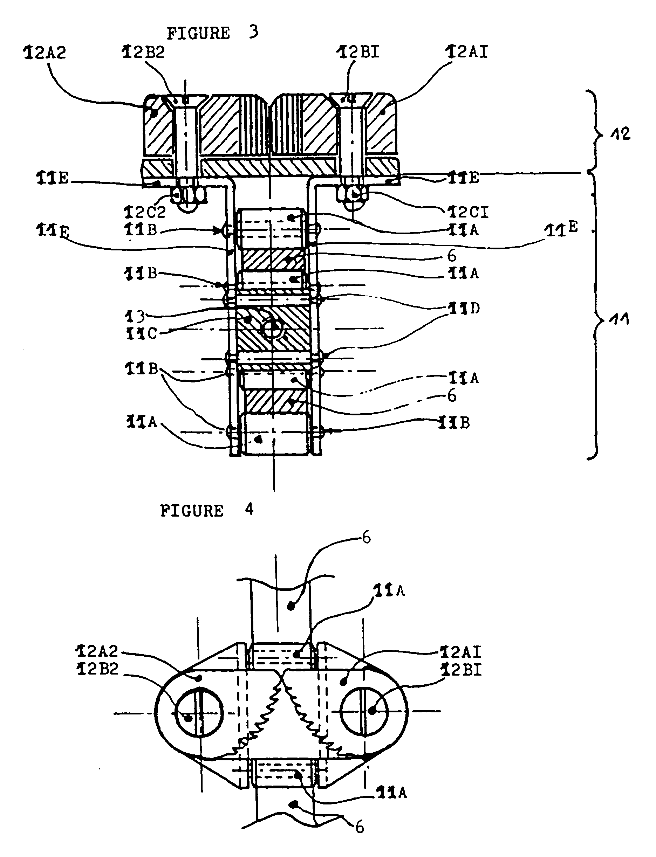

Dans la figure 3 on trouve : Deux flasques équerres 11E, quatre galets 11A, quatre axes 11B, une entretoise 11C, deux rivets 11D, un taquet auto-coinçeur 12 comprenant deux cames crantées 12A1 et 12A2, deux vis pivots 12B1 et 12B2, deux écrous borgnes 12C1 et 12C2, une tige de poussée 13. Cette figure montre également, en coupe, le bâti glissière 6.

Dans la figure 4 on trouve : le bâti glissière 6, non complètement représenté, deux des galets 11A, les deux cames crantées 12A1 et 12A2, les deux vis 12B1 et 12B2.

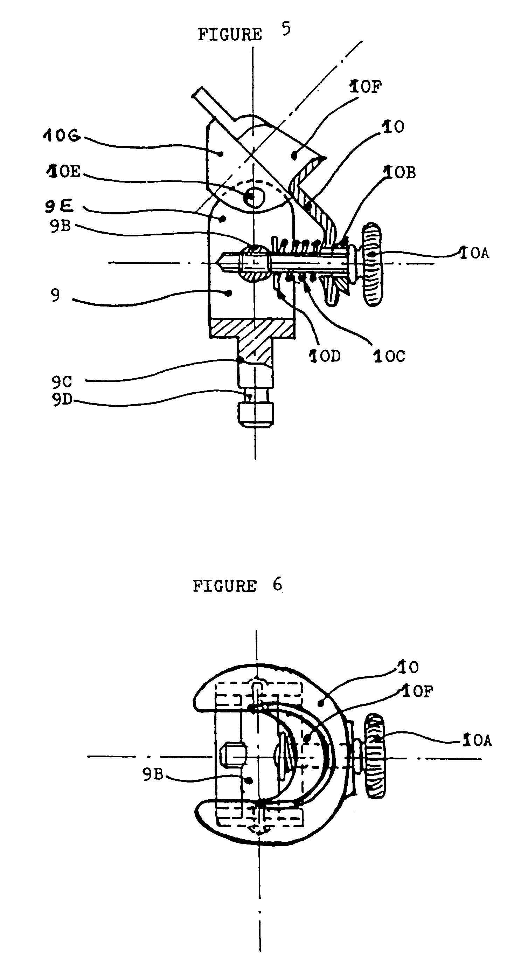

Dans la figure 5 on trouve : la tourelle porte butée 9, un écrou transversal 9B, un tourillon cylindrique 9C doté d'une gorge annulaire 9D, deux oreilles 9E, la butée orientable 10 comportant une collerette de centrage 10 F, la vis 10A, une rondelle bombée 10B, un ressort 10C, une rondelle plate 10D, deux axes 10E, deux flasques 10G.

Dans la figure 6 on trouve : la butée orientable 10, la vis 10A, la collerette 10F, l'écrou transversal 9B.

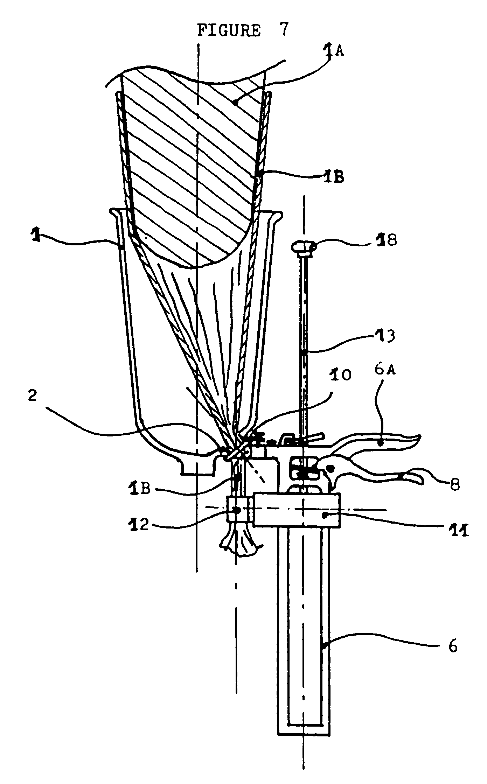

Dans la figure 7 on trouve : l'emboîture de prothèse 1, le fourreau de jersey 1B, le moignon 1A, l'orifice de la soupape 2. Ces éléments nécessaires pour la compréhension du fonctionnement du dispositif ne font pas partie de l'invention. En regard de ceux-ci on trouve une vue simplifiée d'ensemble du dispositif où les éléments suivants sont visibles : le bâti glissière 6, la poignée fixe 6A, la poignée mobile 8, la butée orientable 10, le chariot de traction 11, le taquet autocoinçeur 12, la tige de poussée 13 et le bouton de rappel 18.

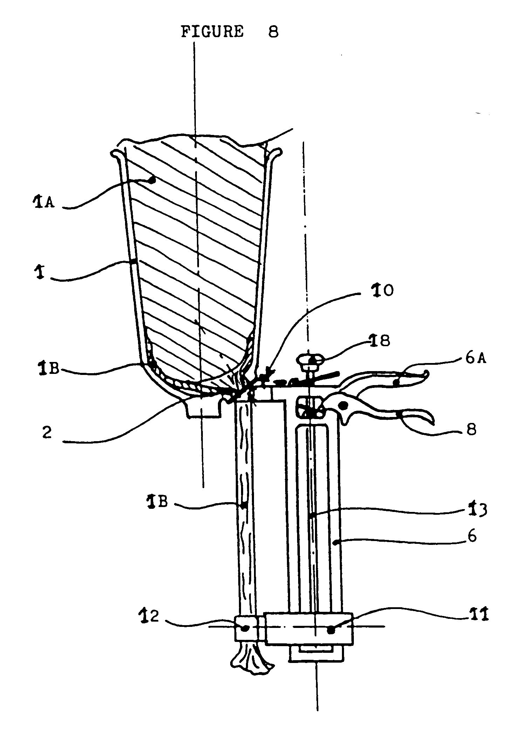

Dans la figure 8, on trouve les mêmes éléments que sur la figure 7.

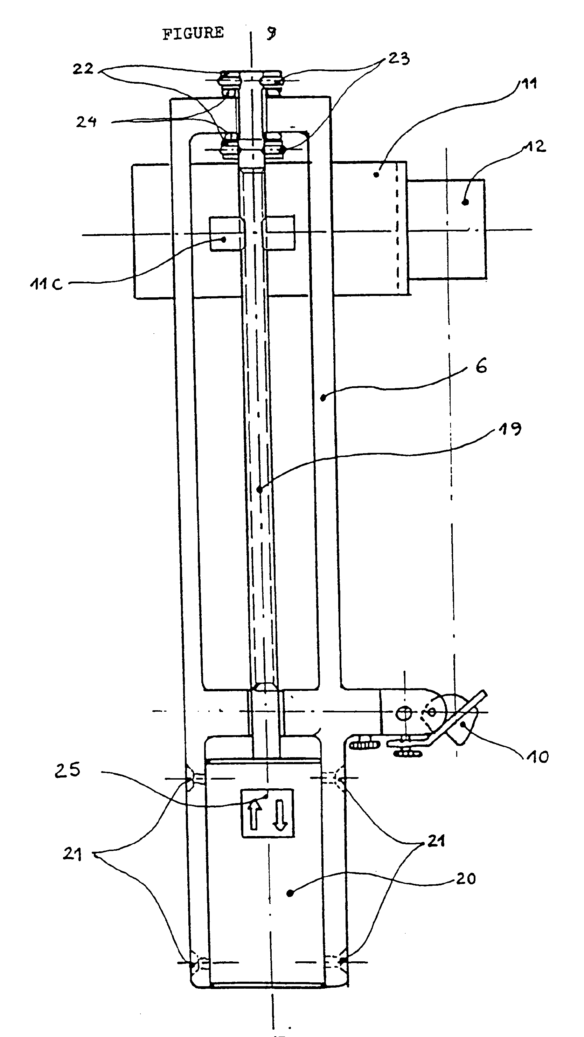

Dans la figure 9, on trouve : le bâti glissière 6, la butée orientable 10, le chariot de traction 11, l'entretoise 11C, le taquet autocoinçeur 12, une vis sans fin 19, un moteur électrique 20, quatre vis de fixation du moteur 21, deux couronnes circulaires de butées de vis sans fin 22, deux goupilles coniques 23, deux rondelles plates antifriction 24 et un interrupteur-inverseur 25.

A présent, l'invention va être décrite en regard des dessins annexés. La figure 1, donnée pour information, ne donne pas lieu à une description plus détaillée.

Dans la figure 2, le bâti glissière 6, la poignée fixe 6A, et la crapaudine 6B peuvent constituer une seule pièce réalisée en alliage léger par injection ou moulage. La poignée mobile 8 pourra être réalisée de la même façon. La tige de poussée 13 sera en acier doux ou mi-dur, non traité. Les ressorts 14 et 16 sont des ressorts à boudin classiques travaillant ici en compression. Les linguets 15 et 17 sont réalisés en acier et sont trempés après usinage pour présenter une bonne résistance à l'usure par frottement. Ces linguets sont sensiblement rectangulaires, dans le plan perpendiculaire au plan de coupe de cette figure 2 et sont forés chacun d'un trou légèrement plus grand que le diamètre de la tige de poussée 13 de façon à ce que celle-ci puisse y glisser librement quand les linguets sont exactement dans un plan perpendiculaire à son axe. Sur cette figure 2 on note que le linguet de retenue 17 se trouve en position oblique par rapport à l'axe de la tige 13 et que de ce fait, celle-ci ne peut reculer. Par contre celle-ci pourrait avancer puisque l'entraînement par frottement du linguet 17 ferait pivoter celui-ci en comprimant le ressort 16. La manoeuvre de la poignée mobile 8 par pivotement autour de son axe 7 entraîne en avant le linguet de poussée 15 qui, retenu par le ressort 14 va se mettre en position oblique et entraîner ainsi la tige de poussée 13 en avant. Ce mécanisme est bien connu et ne sera pas décrit davantage. Il faut savoir qu'à chaque manoeuvre de la poignée 8, la tige 13 avance d'un pas. En fin de course avant, l'opérateur saisi le bouton 18 et le tire en arrière, en tenant dans la main uniquement la poignée fixe 6A et en pressant simultanément, avec le pouce sur la partie la plus longue du linguet 17. Cette opération permet de ramener complètement en arrière la tige de poussée 13. Celle-ci est vissée dans l'entretoise 11C du chariot 11 dont elle est solidaire par les rivets 11D qui passent au travers des flasques 11E. Le chariot de traction 11 se trouve donc solidaire de la tige de poussée 13 et se déplace avec celle-ci.

La conception du chariot de traction 11 s'explique en regard des figures 2, 3 et 4. Le chariot 11 est constitué par les deux flasques équerres 11E, reliés entr'eux par les rivets 11D de l'entretoise 11C. Celle-ci est légèrement plus épaisse que la largeur du bâti glissière 6, de façon à ce qu'il subsiste un jeu suffisant entre la face interne des flasques équerres 11E et les flancs des glissières du bâti 6 pour que le chariot 11 puisse y rester mobile tout en étant guidé. Les huit galets 11A peuvent être réalisés par tournage dans un plastique technique de bonne résistance et ayant de bonnes caractéristiques anti friction, leur épaisseur sera légèrement inférieure à celle de l'entretoise 11C de façon à ce qu'ils puissent tourner librement entre les flasques équerres 11E, sur les axes 11B. L'alésage des galets 11A sera légèrement supérieur au diamètre des axes 11B pour qu'ils puissent tourner librement sur ceux-ci. Les axes 11B auront une longueur totale égale à l'épaisseur de l'entretoise 11C, plus deux fois l'épaisseur d'une flasque 11E plus deux fois la longueur nécessaire pour effectuer un rivetage à l'extérieur des flasques 11E. La partie de ces axes 11B, passant au travers des flasques 11E, aura un diamètre inférieur à celui de la partie centrale, de façon à ce que, au moment du rivetage des axes 11B sur les flasques 11E, la partie centrale des axes 11B serve également d'entretoise aux deux flasques 11E.

Le taquet auto-coinçeur 12 est fixé par les vis 12B1 et 12B2, sur la partie repliée à l'équerre des flasques 11E. Ce taquet auto-coinçeur à rappel automatique par des ressorts qui appuient les cames crantées 12A1 et 12A2, sur des butées internes, non représentées, est d'un modèle bien connu et ne sera pas décrit davantage.

La conception de la butée orientable 10 s'explique en regard des figures 5 et 6. La butée proprement dite 10 peut être réalisée par matriçage dans un métal ductile gardant une bonne résistance après écrouissage. Elle a un diamètre extérieur sensiblement égal à celui du méplat 3 de la figure 1 et est destinée à s'appliquer sur celui-ci. La collerette 10F a un diamètre extérieur légèrement inférieur au diamètre de l'orifice de la soupape 2 de la figure 1, et est destinée à centrer la butée 10 sur le méplat 3, en pénétrant à l'intérieur de l'orifice 2. La butée 10 comporte deux flasques 10G qui sont de part et d'autre de la butée et à l'opposé de la collerette 10F de façon à former une chape qui s'articule, par l'intermédiaire des deux axes 10E, sur les oreilles 9E de la tourelle porte butée 9. La disposition de la vis moletée 10A, de l'écrou transversal 9B, de la rondelle bombée 10B, du ressort 10C (qui travaille en compression), et de la rondelle plate 10D permet par vissage ou dévissage de la vis 10A de faire varier l'inclinaison de la butée orientable 10. La tourelle porte butée 9 grâce à son tourillon 9C qui se positionne dans le trou de la crapaudine porte butée 6B (visible sur la figure 2) permet d'orienter en azimut la butée 10, en desserrant la vis 9A (visible sur la figure 2), en agissant manuellement sur la tourelle porte butée 9, une fois l'orientation désirée obtenue on bloque à nouveau la vis 9A ce qui maintient le réglage désiré. Cette possibilité d'orienter la butée 10, en site et en azimut, permet de pouvoir utiliser le dispositif, objet de la présente invention, sur des emboîtures de prothèse ayant des orientations différentes de l'orifice 2 de la soupape. A noter que la butée 10 et la collerette 10F sont ouvertes et s'apparentent à une fourchette à deux dents, ce qui permet d'enfourcher la butée 10 sur le fourreau de jersey, sans nécessiter d'enfiler ce dernier dans la butée 10.

La description de l'utilisation du dispositif est illustrée par les figure 7 et 8. Sur la figure 7 qui représente le dispositif en début d'opération, on voit qu'un fourreau de jersey 1B a été enfilé sur le moignon 1A, puis introduit, par l'intérieur de l'emboîture de la prothèse, au travers de l'orifice de la soupape 2. Le dispositif a été mis en place contre l'emboîture de la prothèse 1, et prend appui sur le méplat de l'orifice 2 par l'intermédiaire de la butée orientable 10. La partie libre du fourreau de jersey 1B, à l'extérieur de l'emboîture 1, passe au travers du passage de la butée 10 et a été coincée dans le taquet auto-coinçeur 12, solidaire du chariot de traction 11 qui se trouve en position arrière, la tige de poussée 13 ayant été tirée au préalable en arrière par le bouton 18. A partir de cet état, le dispositif est prêt pour entrer en action. L'opérateur presse alors la poignée mobile 8 contre la poignée fixe 6A, ce qui a pour effet de faire avancer d'un pas la tige de poussée 13, ce qui fait avancer d'autant le chariot de traction 11 et le taquet auto-coinçeur 12. Celui-ci tire sur le fourreau de jersey 1B, la réaction de cette force de traction repousse l'emboîture 1 vers le moignon 1A. L'opérateur répète autant de fois que nécessaire sa pression sur la poignée mobile 8, jusqu'à ce que le dispositif se trouve dans la configuration représentée sur la figure 8. Si le chariot de traction 11, atteint la fin de sa course maximum, avant que le fourreau de jersey 1B soit complètement dégagé de l'emboîture 1, il suffit à l'opérateur de décoincer le fourreau de jersey 1B du taquet auto-coinçeur 12, de remettre le dispositif en position de départ, tel que représenté sur la figure 7, puis de recoincer le fourreau de jersey 1B dans le taquet auto-coinçeur 12 et recommencer la traction.

La figure 9 représente une variante de réalisation du dispositif, qui fonctionne de la manière suivante : le moteur 20, similaire à ceux des tournevis électriques, fonctionnant de préférence sur accumulateurs, entraîne la vis sans fin 19, en rotation dans un sens ou dans l'autre selon la position donnée par l'opérateur à l'interrupteur inverseur 25. Cela a pour effet de déplacer le chariot de traction dans un sens ou dans l'autre et de conduire au même résultat qu'avec le dispositif dans sa version manuelle. A noter que les couronnes de butée circulaires 22, montées de part et d'autre de l'extrémité du bâti glissière 6, servent de point d'appui à la vis sans fin, ce qui présente l'avantage de faire travailler celle-ci en traction quand le chariot de traction 11 se déplace dans le sens où il tire effectivement sur le fourreau de jersey 1B. Cela produit simultanément la mise en compression du bâti glissière 6 dont le métal coulé par injection est mieux adapté à ce type de contrainte.

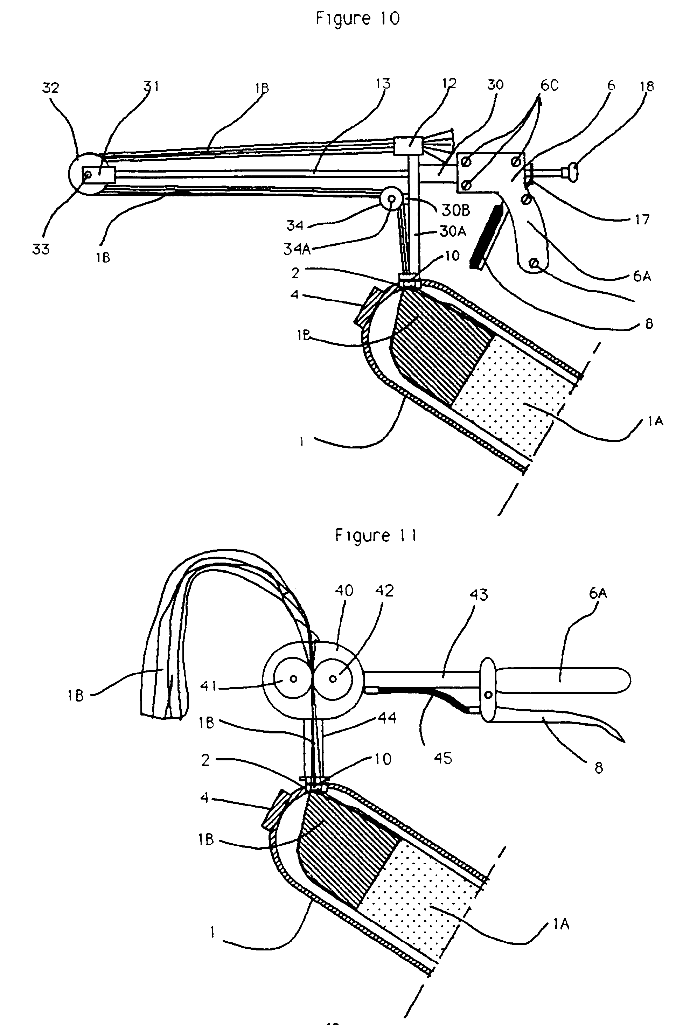

Dans la figure 10 on trouve : L'emboîture de prothèse 1 et le moignon 1A (interrompus), le fourreau de jersey 1B, l'orifice de soupape d'extraction d'air 2, le plan de pose 4, le bâti glissière 6, la poignée fixe 6A, cinq vis 6C, la poignée mobile 8, la butée 10, le taquet coinçeur 12, la tige de poussée 13, le linguet de retenue 17, le bouton de rappel 18, une entretoise 30, une traverse 30A, un doigt excentré 30B, une chape 31, un réa 32, un axe 33, un réa de renvoi 34, et un axe de réa de renvoi 34A.

Dans cette première variante, le bâti glissière 6 est raccourci et est, dans ce cas, réalisé en deux demi coquilles symétriques, assemblées par les vis 6C. La tige de poussée est beaucoup plus longue que le bâti glissière 6. Cette tige 13, à son extrémité opposée au bouton de rappel 18, est solidaire d'une chape 31 qui porte un réa à gorge 32, réalisé dans un matériau léger et qui tourne sur l'axe 33 porté par la chape 31. Ce réa 32 à une gorge circulaire périphérique en forme de demi-cercle, pour recevoir et guider le fourreau de jersey 1B. L'entretoise orientable 30 est tubulaire et de section cylindrique et est réalisée, par décolletage au tour, dans un matériau léger. Elle est retenue dans le bâti glissière, par deux demi coussinets (un par demi coquille), qui sont concentriques avec l'axe de la tige de poussée 13. Cette entretoise 30 peut tourner dans les deux demi-coussinets quand les vis 6C sont légèrement desserrées et elle se trouve bloquée en position quand ces mêmes vis 6C sont serrées à fond. L'entretoise 30 est forée axialement par un trou de section circulaire pour permettre le passage de la tige de poussée 13. L'entretoise 30, est solidaire de la traverse 30A qui porte, à son extrémité supérieure, le taquet coinçeur 12, et à son extrémité inférieure la butée 10. La traverse 30A porte le doigt excentré 30B qui porte, en porte à faux, l'axe 34A sur lequel tourne le réa de renvoi 34. La traverse 30a est percée d'un trou de même diamètre que celui de l'entretoise 30 et en regard de celui-ci. Le réa 34 est identique, dans son principe, au réa 32. Dans cette variante, le mécanisme de poussée est contenu à l'intérieur des deux demi coquilles qui forment le bâti glissière 6 et n'est donc pas visible. Il est semblable, en son principe, à celui décrit en regard de la figure 2 et, à chaque manoeuvre de la poignée mobile 8 par rapport à la poignée fixe 6A, la tige de poussée 13 avance d'un pas.

Le fonctionnement du dispositif, selon cette première variante, est le suivant : en premier lieu, la tige de poussée 13 est ramenée en position arrière en tirant le bouton de rappel 18. Cette manoeuvre à pour effet d'amener en butée la chape 31 contre la traverse 30A. L'opérateur, après avoir enfilé le fourreau de jersey 1B sur le moignon 1A et après avoir enfilé la partie libre du fourreau de jersey 1B dans l'orifice 2, en passant par l'intérieur de l'emboîture de la prothèse 1, positionne la butée 10 sur l'orifice 2 et passe successivement le fourreau de jersey 1B dans la gorge du réa 34, puis dans la gorge du réa 32 pour le fixer dans le taquet coinçeur 12. Ensuite l'opérateur presse la poignée mobile 8 contre la poignée fixe 6A, ce qui a pour effet d'éloigner la chape 31 de la traverse 30A, donc de tirer sur le fourreau de jersey 1B ce qui fait pénétrer le moignon 1A dans l'emboîture 1. Cette opération est répétée autant de fois que nécessaire pour qu'il y ait extraction complète du fourreau de jersey 1B de l'emboîture 1.

Dans la figure 11 on trouve : l'emboîture de prothèse 1 et le moignon 1A (interrompus), le fourreau de jersey 1B, l'orifice de soupape d'extraction d'air 2, le plan de pose 4, la poignée fixe 6A, la poignée mobile 8, la butée 10, un carter 40, deux rouleaux 41, 42, un tube 43, une traverse 44 et une gaine de câble 45.

L'ensemble formé par la poignée fixe 6A, la poignée mobile 8, le tube 43 et la gaine de câble 45 est tout à fait semblable à un demi guidon de bicyclette et peut être réalisé de la même façon, à savoir : le tube 43 porte la poignée 6A ainsi que la poignée 8, l'une des extrémités de la gaine de câble 45 est en butée sur la partie fixe de la poignée mobile 8, et son autre extrémité est en butée sur le carter 40, elle contient un câble, non représenté, qui commande le mécanisme de manoeuvre des rouleaux 41 et 42. La poignée 8 peut être orientée autour du tube 43 et fixée en position, après réglage, comme cela se pratique pour une poignée de commande de frein de bicyclette. Le tube 43 est solidaire du carter 40. Celui-ci peut être réalisé en alliage léger moulé ou injecté, il porte le mécanisme d'entraînement des deux rouleaux 41 et 42. Ce mécanisme d'entraînement a deux fonctions :
1) il fait tourner, du même angle, les rouleaux 41 et 42 à chaque pression sur la poignée 8, mais en sens contraire l'un par rapport à l'autre, c'est à dire que le rouleau 41 tourne d'un angle donné dans le sens anti horaire alors que simultanément le rouleau 42 tourne d'un angle de même valeur mais dans le sens horaire. Les deux rouleaux sont dotés de mécanismes genre "roue libre" qui n'autorise leur rotation que comme décrite précédemment en les empêchant de repartir en arrière.
2) il appuie l'un contre l'autre les rouleaux 41 et 42. Une manette non représentée permet d'écarter l'un de l'autre les deux rouleaux 41 et 42. La traverse 44 est solidaire du carter 40, elle est réalisée dans un tube ou un profilé non complètement fermé et elle porte, à son extrémité inférieure, la butée 10 dont elle est solidaire.

Le fonctionnement du dispositif, selon cette deuxième variante, est le suivant : après avoir, comme précédemment, enfilé le fourreau de jersey 1B sur le moignon 1A et après avoir enfilé la partie libre du fourreau de jersey 1B dans l'orifice 2, en passant par l'intérieur de l'emboîture de la prothèse 1, l'opérateur positionne la butée 10 sur l'orifice 2 et il passe le fourreau de jersey 1B entre les rouleaux 41 et 42 en ayant, au préalable, écarté ceux-ci par la manoeuvre de la manette non représentée, puis il relâche celle-ci pour que les rouleaux 41 et 42 pressent le fourreau de jersey. Ensuite l'opérateur presse la poignée 8 contre la poignée 6A, autant de fois que nécessaire pour que l'entraînement du fourreau de jersey, par la rotation inverse des deux rouleaux 41 et 42 ait complètement extrait celui-ci de l'emboîture de la prothèse 1.

### AVANTAGE RESULTANT DE LA PRESENTE INVENTION

On va récapituler ci-après les avantages de la présente invention.
- Dans sa version de base, à commande manuelle, le dispositif requiert un effort de pression de la main de l'opérateur, quatre à six fois moins élevé que celui nécessité par la traction manuelle directe, sur le fourreau de jersey 1B. De plus, cette pression de la main, qui s'exerce par simple fermeture des doigts sur la poignée à deux branches 8 et 6A ne sert qu'à générer l'effort de traction, l'effort de préhension du fourreau de jersey étant supprimé et assumé par le taquet auto-coinçeur 12.
- La position de la poignée de manoeuvre 8 et 6A est, au point de vue ergonomique, idéale, aussi bien pour l'amputé lui-même que pour une tierce personne.
- Le dispositif assure simultanément, la traction sur le fourreau de jersey 1B, et le guidage de l'emboîture de la prothèse 1 vers le moignon 1A, en repoussant celle-ci vers celui-là.
- Dans sa version de base, à commande manuelle, le dispositif est de type "sensitif", en ce que l'opérateur peut apprécier, selon l'effort qui'il doit exercer sur la poignée 8 et 6A, l'intensité du coincement du fourreau de jersey 1B, entre l'intérieur de l'emboîture 1 et le moignon 1A et d'y remédier, en cas de coincement excessif, en ne prenant que partiellement le fourreau de jersey 1B, dans le taquet auto-coinçeur 12, ou en faisant remuer le moignon 1A par de petits mouvements circulaires, favorables au décoincement du fourreau 1B. A noter que ce dernier reste en tension par sa propre élasticité en cas d'arrêt de la traction en cours d'opération, et que de ce fait le fourreau se décoince et avance seul dès que les petits mouvements circulaires, mentionnés ci-avant, le libèrent, et cela, sans qu'il y ait nécessité de manoeuvrer simultanément la poignée 8 et 6A. L'opérateur n'a donc à se préoccuper que d'une seule chose à la fois.
- La préhension du fourreau de jersey 1B, dans le taquet auto-coinçeur 12 est des plus aisée et s'opère latéralement, (au passage), de la même manière que pour coincer les écoutes sur les embarcations à voiles. Il est possible de ne coincer qu'une partie du périmètre du fourreau de jersey 1B, comme cela est d'usage dans la pratique courante.
- Compte tenu de cette facilité et rapidité de préhension du fourreau de jersey dans le taquet auto-coinçeur, il est possible de réaliser des dispositifs ayant un bâti glissière 6 court, donc une course réduite, et d'effectuer l'opération en plusieurs tirages successifs. On pourrait réaliser ainsi des dispositifs peu encombrants et très légers, pour les amputés qui ne mettent en place leur prothèse qu'une seule fois par jour, et réaliser des dispositifs plus longs, permettant le tirage du fourreau en une seule fois, pour les usages intensifs dans les établissements spécialisés.
- Grande facilité de mise en place du dispositif sur l'orifice de la soupape 2 de l'emboîture 1 de la prothèse, après que le fourreau de jersey 1B ait été passé au travers de cet orifice 2 et sans nécessiter l'enfilage du fourreau de jersey 1B dans la butée 10 du fait que celle-ci est ouverte et se présente comme une sorte de fourchette à deux dents qu'il suffit d'enfourcher sur le fourreau de jersey 1B, au niveau de sa sortie, à l'extérieur de l'orifice 2 de la soupape de l'emboîture de la prothèse 1, avant d'engager la collerette 10F dans cet orifice 2.
- Butée 10 orientable en site et en azimut, ce qui permet d'utiliser le dispositif sur des prothèses dont les orifices de soupape sont orientés différemment.
- Dans sa version à moteur électrique, fonctionnant sur accumulateurs, du même type que ceux des tournevis électriques, l'effort manuel est supprimé, mais l'effet sensitif est moindre. Toutefois il faut noter que ces types de moteurs ont des couples limités et que l'opérateur, avec un peu d'habitude, peut également apprécier l'état de coincement du fourreau de jersey 1B selon la vitesse du moteur 20 et la variation du son émis par celui-ci durant son fonctionnement. Cette conception peut être réservée aux amputés ayant des difficultés pour produire des efforts manuels, même très légers.

Les avantages résultant de cette variante visible sur la figure 10 sont les suivants :
- A chaque pression sur la poignée 8, le fourreau de jersey 1B est allongé de deux fois la dimension du pas d'avance de la tige de poussée 13, ce qui fait que cette dernière, à longueur de tirage égale pour le fourreau de jersey 1B, sera deux fois moins longue.
- Le bâti glissière 6, peut pivoter de 360 degrés autour de l'entretoise 30, ce qui a pour effet de rendre ce dispositif adaptable à toutes les morphologies, tant pour le handicapé lui-même ou pour une tierce personne qui l'aide à s'appareiller.
- La présence du réa de renvoi 34, change l'orientation de la réaction de la force de traction au niveau de la butée 10 et de l'orifice 2 autour duquel elle prend appui. En effet il n'y a pratiquement pas de composante horizontale au niveau de l'orifice 2 et la butée 10 est poussée sensiblement suivant l'axe de cet orifice 2.

Les avantages résultant de la deuxième variante visible sur la figure 11, sont les suivants :
- Le dispositif complet peut être très court puisque la tige de poussée 13 est supprimée.
- En fin d'opération le dispositif n'a pas à être remis en position de départ puisque son fonctionnement résulte d'une rotation.
- Il met en oeuvre des moyens connus et éprouvés à des millions d'exemplaires sur les bicyclettes.

## Revendications

1. Dispositif de traction sur les fourreaux de jersey, pour mise en place des prothèses de contact, lesquelles comprennent une emboîture (1) fermée à l'une de ses extrémités sur laquelle est prévu un orifice d'une soupape d'extraction d'air (2), ledit dispositif comportant une butée (10), caractérisé en ce que cette butée (10) est adaptée à prendre appui sur ladite extrémité fermée de l'emboîture (1) de la prothèse, au voisinage dudit orifice de la soupape d'extraction d'air (2), sur l'emboîture de façon que la réaction de la force de traction s'exerce sur ce point d'appui de la butée sur ladite extrémité.

2. Dispositif selon revendication 1), caractérisé en ce qu'il comporte un taquet auto-coinçeur (12), et en ce que ce taquet (12) est adapté à retenir fermement le fourreau de jersey sur la totalité de sa section ou seulement sur une partie de celle-ci.

3. Dispositif selon revendication 1), caractérisé en ce qu'il comporte un chariot de traction (11) solidaire du taquet (12) et en ce que le chariot de traction (11) se déplace le long d'un bâti-glissière (6) et en ce que la butée (10) est solidaire du bâti-glissière (6).

4. Dispositif selon revendication 1) ou 3), caractérisé en ce que le moyen de déplacement du chariot de traction (11) le long du bâti-glissière (6) est un système de poussée (13, 14, 15, 16, 17, 18), et en ce que le dit système de poussée est commandé par une poignée à deux branches (8, 6A), et en ce que la branche fixe (6A) de cette poignée (8, 6A) est solidaire du bâti-glissière (6) et reste donc fixe par rapport à la butée (10).

5. Dispositif selon l'une quelconque des revendications précédentes, caractérisé en ce que la butée (10) est réglable, en Site et en Azimut, par des moyens (10A, 10B, 10C, 10D, 10E, 10G, 9E, 9B, 9C, 9D et 9A).

6. Dispositif selon l'une quelconque des revendications précédentes caractérisé en ce que la butée (10) comporte une collerette (10F) adaptée à centrer celle-ci autour de l'orifice (2) en pénétrant à l'intérieur de ce dernier, et en ce que la butée (10) ainsi que-la collerette (10F) sont ouvertes de façon à former une fourche à deux dents, permettant de mettre la butée (10) en place sur l'orifice (2), sans être obligé d'enfiler le fourreau de jersey (1B) dans l'orifice de la butée (10).

7. Dispositif selon l'une quelconque des revendications 1, 2, 3, 5 ou 6, caractérisé en ce que le système de poussée du chariot de traction (11) est une vis sans fin (19) actionnée par un moteur électrique (20) à deux sens de marche, commandé par un interrupteur poussoir inverseur (25).

8. Dispositif selon l'une quelconque des revendications 1) à 7), caractérisé en ce que le moteur (20) est un moteur électrique fonctionnant sur une batterie d'accumulateurs, incorporée au moteur (20), et rechargeable sur un bloc chargeur mural, servant de râtelier sur lequel on embroche l'ensemble du dispositif pour à la fois le charger et le ranger, en dehors des périodes d'utilisation.

9. Dispositif selon l'une quelconque revendications précédentes caractérisé en ce que le chariot de traction (11) comporte des galets de roulement (11A) qui limitent son frottement sur le bâti-glissière (6).

10. Dispositif selon revendications 1, 2, 5, 6, caractérisé en ce que la tige de poussée (13) est plus longue que le bâti (6) et porte une chape (31) dans laquelle tourne un réa à gorge (32).

11. Dispositif selon revendication 10, caractérisé en ce que le taquet coinçeur (12) ainsi que la butée (10) sont solidaires d'une traverse (30A) solidaire elle-même d'une entretoise orientable (30) qui est coaxiale avec l'axe de la tige de poussée (13), et en ce que l'entretoise (30 est orientable de O à 360 degrés par rapport au bâti glissière (6) et en ce que l'entretoise (30) peut être bloquée en rotation par rapport au bâti glissière (6) au moyen de vis (6C).

12. Dispositif selon revendications 1, 5 et 6, caractérisé en ce que la traction du fourreau de jersey (1B) est obtenue par la rotation inverse de deux rouleaux (41, 42) qui pincent entre eux deux le fourreau de jersey (1B), et en ce que les rouleaux (41, 42) peuvent être écartés l'un de l'autre au moyen d'une manette, et en ce que le mécanisme qui met en rotation les rouleaux (41, 42) est commandé par la manoeuvre répétée d'une poignée mobile (8) par rapport à une poignée fixe (6A).

## Patentansprüche

1. Zugvorrichtung auf Futteralen aus Jersey zum Anlegen von Kontaktprothesen, die eine Einsteckverbindung (1) aufweisen, die an dem einen ihrer Enden, an dem eine Öffnung eines Luftabzugsventils (2) vorgesehen ist, geschlossen ist, wobei die Vorrichtung einen Anschlag (10) aufweist, dadurch gekennzeichnet, daß der Anschlag (10) so angepaßt ist, daß er auf dem geschlossenen Ende der Einsteckverbindung (1) der Prothese in der Nähe der Öffnung des Luftabzugsventils (2) auf der Einsteckverbindung derart aufliegt, daß die Reaktion der Zugkraft auf diesen Auflagepunkt des Anschlags an dem Ende ausgeübt wird.

2. Vorrichtung gemäß Anspruch 1, dadurch gekennzeichnet, daß sie ein selbst-festklemmendes Einraststück (12) aufweist, und daß dieses Einraststück (12) so angepaßt ist, daß es das Futteral aus Jersey fest auf der Gesamtheit seines Abschnitts oder nur auf einem Teil von diesem festhält.

3. Vorrichtung gemäß Anspruch 1, dadurch gekennzeichnet, daß sie einen mit dem Einraststück (12) einstückig ausgebildeten Zugschlitten (11) aufweist, und daß sich der Zugschlitten (11) entlang eines Laufschienen-Gestells (6) verschiebt, und daß der Anschlag (10) einstückig mit dem Laufschienen-Gestell (6) ausgebildet ist.

4. Vorrichtung gemäß Anspruch 1 oder 3, dadurch gekennzeichnet, daß die Verschiebungseinrichtung des Zugschlittens (11) entlang dem Laufschienen-Gestell (6) ein Schubsystem (13, 14, 15, 16, 17, 18) ist, und daß das Schubsystem von einem Griff mit zwei Armen (8, 6A) gesteuert wird, und daß der feste Arm (6A) dieses Griffes (8, 6A) einstückig mit dem Laufschienen-Gestell (6) ausgebildet ist und somit fest bezüglich des Anschlags (10) bleibt.

5. Vorrichtung gemäß irgendeinem der vorherigen Ansprüche, dadurch gekennzeichnet, daß der Anschlag (10) einstellbar in Site und in Azimut durch Einrichtungen (10A, 10B, 10D, 10E, 10G, 9E, 9B, 9C, 9D und 9A) ist.

6. Vorrichtung gemäß irgendeinem der vorherigen Ansprüche, dadurch gekennzeichnet, daß der Anschlag (10) einen Flansch (10F) aufweist, der so angepaßt ist, daß er den Anschlag um die Öffnung (2) zentriert, wobei er in das Innere dieser letzteren eindringt, und daß der Anschlag (10) sowie der Flansch (10F) offen sind, derart, daß sie eine Gabel mit zwei Zähnen bilden, die ermöglicht, den Anschlag (10) auf der Öffnung (2) anzuordnen, ohne gezwungen zu sein, das Futteral aus Jersey (1B) in die Öffnung des Anschlags (10) einzufädeln.

7. Vorrichtung gemäß irgendeinem der Ansprüche 1, 2, 3, 5 oder 6, dadurch gekennzeichnet, daß das Schubsystem des Zugschlittens (11) eine Schraube ohne Ende (19) ist, die von einem elektrischen Motor (20) mit zwei Laufrichtungen betätigt wird, der von einem Kipp-Druckschalter (25) gesteuert wird.

8. Vorrichtung gemäß irgendeinem der Ansprüche 1 bis 7, dadurch gekennzeichnet, daß der Motor (20) ein elektrischer Motor ist, der mit einer in den Motor (20) eingebauten Akkumulatorenbatterie funktioniert, die auf einem Wandladeblock aufladbar ist, der als Gestell dient, auf dem man die Gesamtheit der Vorrichtung in den Stromkreis schaltet, um sie außerhalb der Benutzungszeiten zugleich aufzuladen und zu ordnen.

9. Vorrichtung gemäß irgendeinem der vorherigen Absprüche, dadurch gekennzeichnet, daß der Zugschlitten (11) Laufrollen (11A) aufweist, die seine Reibung auf dem Laufschienen-Gestell (6) begrenzen.

10. Vorrichtung gemäß den Ansprüchen 1, 2, 5, 6, dadurch gekennzeichnet, daß die Schubstange (13) länger als das Gestell (6) ist und eine Abdeckung (31) trägt, in der sich eine Schnurrolle mit Auskehlung (32) dreht.

11. Vorrichtung gemäß Anspruch 10, dadurch gekennzeichnet, daß das Klemm-Einraststück (12) sowie der Anschlag (10) einstückig mit einem Querträger (30A) ausgebildet sind, der selbst einstückig mit einem verstellbaren Steg (30) ausgebildet ist, der koaxial mit der Achse der Schubstange (13) ist, und daß der Steg (30) verstellbar um 0 bis 360 Grad bezüglich dem Laufschienen-Gestell (6) ist, und daß der Steg (30) in Drehung bezüglich dem Laufschienen-Gestell (6) mittels Schrauben (6C) blockiert werden kann.

12. Vorrichtung gemäß den Ansprüchen 1, 5 und 6, dadurch gekennzeichnet, daß der Zug des Futterals aus Jersey (1B) durch die umgekehrte Drehung von zwei Rollen erreicht wird, die zwischen sich beiden das Jersey-Futteral (1B) einklemmen, und daß die Rollen (41, 42) mittels eines Bedienungshebels in Abstand zueinander liegen können, und daß der Mechanismus, der die Rollen (41, 42) in Drehung setzt, von der wiederholten Bedienung eines beweglichen Griffes (8) bezüglich eines festen Griffes (6A) gesteuert wird.

## Claims

1. Jersey sheath drawing device, for fitting contact prostheses which comprise a nest (1) closed at one end on which an air extraction valve opening (2) is provided, the said device including a bearing (10), characterised in that this bearing (10) is designed to rest on the said closed end of the nest (1) of the prosthesis in the vicinity of the said air extraction valve opening (2) on the nest so that the reaction of the pulling force is exerted on this point at which the bearing rests on the said end.

2. Device according to Claim 1, characterised in that it includes a self-locking catch (12) and in that this catch (12) is designed to grip the jersey sheath tightly over the whole or only a part of its cross-section.

3. Device according to Claim 1, characterised in that it includes a pulling runner (11) fastened to the catch (12) and in that the pulling runner (11) moves along a sliding frame (6) and in that the bearing (10) is fastened to the sliding frame (6).

4. Device according to Claim 1 or 3, characterised in that the means for moving the pulling runner (11) along the sliding frame (6) is a pusher system (13, 14, 15, 16, 17, 18), and in that the said pusher system is controlled by a two-part handle (8, 6A), and in that the fixed part (6A) of this handle (8, 6A) is fastened to the sliding frame (6) and thus remains fixed relative to the bearing (10).

5. Device according to any one of the preceding claims, characterised in that the bearing (10) is adjustable in elevation and azimuth by means (10A, 10B, 10C, 10D, 10E, 10G, 9E, 9B, 9C, 9D and 9A).

6. Device according to any one of the preceding claims, characterised in that the bearing (10) includes a flange (10F) designed to centre it around the opening (2) by entering the latter, and in that the bearing (10) and the flange (10F) are open so as to form a two-pronged fork enabling the bearing (10) to be fitted over the opening (2) without the need to thread the jersey sheath (1B) into the opening of the bearing (10).

7. Device according to any one of Claims 1, 2, 3, 5 or 6, characterised in that the pusher system of the pulling runner (11) is a worm (19) driven by a reversible electric motor (20) controlled by a push-button reversing switch (25).

8. Device according to any one of Claims 1 to 7, characterised in that the motor (20) is an electric motor powered by a battery incorporated in the motor (20) and rechargeable on a wall-mounted charging unit serving as a rack into which the whole device is plugged in order both to charge it and to stow it away when not in use.

9. Device according to any one of the preceding claims, characterised in that the pulling runner (11) includes rollers (11A) which limit its friction against the sliding frame (6).

10. Device according to Claims 1, 2, 5, 6, characterised in that the push rod (13) is longer than the frame (6) and carries a yoke (31) in which a grooved pulley wheel (32) rotates.

11. Device according to Claim 10, characterised in that the locking catch (12) and the bearing (10) are fastened to a transverse member (30A) itself fastened to an adjustable spacer (30) which is coaxial with the axis of the push rod (13), and in that the spacer (30) can be adjusted from 0 to 360° relative to the sliding frame (6) and in that the spacer (30) may be prevented from rotating relative to the sliding frame (6) by means of screws (6C).

12. Device according to Claims 1, 5 and 6, characterised in that the jersey sheath (1B) is pulled by the rotation, in opposite directions, of two rollers (41, 42) which grip the jersey sheath (1B) between them, and in that the rollers (41, 42) may be separated from each other by means of a handle, and in that the mechanism which rotates the rollers (41, 42) is controlled by the repeated actuation of a moving handle (8) relative to a fixed handle (6A).
